# EUROPEAN PATENT SPECIFICATION

(11) **EP 2 559 432 B1**
(45) Date of publication and mention of the grant of the patent: **26.06.2019**
(21) Application number: 10849929.4
(22) Date of filing: 11.11.2010
(51) Int. Cl.: A61K 31/045, A61P 1/18, A61K 33/42, A61K 31/661

(54) **MEANS FOR THE PROPHYLAXIS AND TREATMENT OF ACUTE AND CHRONIC PANCREATITIS**
MITTEL ZUR PROPHYLAXE UND BEHANDLUNG VON AKUTER UND CHRONISCHER PANKREATITIS
MOYEN POUR LA PRÉVENTION ET LE TRAITEMENT DE PANCRÉATITE AIGUË ET CHRONIQUE

(30) Priority: 13.04.2010 RU 2010114558
(43) Date of publication of application: 20.02.2013
(73) Proprietor: Limited Liability Company «GamaVetFarm», Moscow 123098 (RU)
(72) Inventor: NAROVLYANSKIJ, Alexandr Naumovich, Moscow 125171 (RU); PRONIN, Alexander Vasil'evich, Moscow 105554 (RU); SANIN, Alexander Vladimirovich, Moscow 125445 (RU); TRUBITSYNA, lrina Evgen'evna, Moscow 103009 (RU)
(74) Representative: von Füner, Nicolai
(86) International application number: PCT/RU2010/000663
(87) International publication number: WO 2011/129716

(56) References cited:
- EP-A1- 0 548 825
- EP-A1- 0 699 440
- EP-A2- 0 350 801
- EP-A2- 0 350 801
- RU-A- 2007 123 141
- RU-C1- 2 005 475
- RU-C2- 2 177 788
- RU-C2- 2 354 350
- US-A- 4 613 593
- US-B1- 6 525 035
- MIKRO-PLYUS.: 'Lekarstva dlya zhyvotnykh.' NASTAVLENIE PO PRIMENTNIYU FOSPRENILA PRI VIRUSNYKH INFEKTSIYAKH ZHYVOTNYKH., [Online] 03 June 2003 - 01 July 2011, XP008158230 Retrieved from the Internet: <URL:http:www.micro-plus.ru/fos_dir.htm> [retrieved on 2011-07-01]
- OKHLOBYSTIN ET AL.: 'Soveremennye vozmoshnosti terapii khronicheskogo pankreatita.' LECHASCHI VRACH: ZHURNAL DLYA PRAKTIKUYUSCHEGO VRACHA, [Online] no. 5, 2003, pages 32 - 36, XP008158235 Retrieved from the Internet: <URL:http://www.lvrach.ru/2003/05/4530306/? format=print> [retrieved on 2011-06-06]

## Description

### Field of invention

The invention relates to drugs for use in the prophylaxis and treatment of acute and chronic pancreatitis.

### Prior art

Drugs for the treatment of acute and chronic pancreatitis, such as phosphalugel, almagel, Bourget mixture, cimetidine, sandostatin, omeprasol, contrical are known (Okhlobystin A.V., Buklis E.R. Current possibilities of chronic pancreatitis therapy // Attending Physician: Journal for General Practitioner. 2003. N 5. P. 32-36; Samsonov A.A. Advanced pharmacotherapy of chronic pancreatitis // Russian Pharmacy. 2007. N 3. P. 30-32).

US 6,525,035 B1 discloses pharmaceutical compositions comprising isolated polyprenol monophosphates and isolated polyprenol pyrophosphates or isolated polyprenol pyrophosphates alone and teaches that the invention provides a method for producing an antiviral effect, for modulating the immune system, for the treatment of several diseases caused by e.g. a distemper virus, for upregulating the Th1 system and for enhancing the protective effects of a vaccine.

EP 0 350 801 A2 discloses a composition comprising one or more polyprenols and polyprenyl phosphates for the inhibition of tumor metastasis in mammals.

US 4,613,593 discloses a method for treating hyperlipidemia or arteriosclerosis which comprises administering to a subject a composition comprising *inter alia* dolichol , a group of long-chain organic compounds with varying numbers of isoprene units, especially dolichol phosphate, dolichol dipolyphosphate and dolichol tripolyphosphate.

EP 0 699 440 A1 discloses a therapeutic composition for pancreatitis which comprises ascorbyl tocopheryl phosphate, which is a diester of tocopherol (vitamin E) and ascorbic acid (vitamin C).

The known drugs can only eliminate symptoms of the disease and are not used for prophylaxis.

### Disclosure of the invention

The technical result of using the claimed drug for use in the prophylaxis and treatment of acute and chronic pancreatitis consists in the elimination of inflammation and stimulation of the regeneration of pancreas cells.

Said technical result is achieved due to the fact that the drug for prophylaxis and treatment of acute and chronic pancreatitis contains a mixture of different polyprenyl phosphates and polyprenyl pyrophosphates with 7 to 30 isoprene unitsin the mass ratio from 5:1 to 20:1, preferably 9:1.

### Preferable embodiment

The new drug is administered intramuscularly, intravenously, intraperitoneally, orally, or by direct treatment of the pancreas during an operation. The drug can be administered in dry and liquid forms (tablets, capsules, suppositories, solutions for injections).

The drug is usually introduced 5 times in a dose corresponding to 10 (5-20) mg of active substance per 1 kg of body mass once a day (or once in two days).

The drug was tested and proved efficient.

The experiments were carried out on white rats weighing 180-220 g. The tissue of the pancreas was damaged by acetic acid (0.025 ml), which was applied on the pancreas surface.

### Test pattern:

The acute phase: introduction (intraperitoneal) from the first day of the reproduction of pancreatitis during 5 days.

The chronic phase: introduction (intraperitoneal) - on the first, third, fifth, seventh, ninth day.

The experiment was performed on two groups (acute and chronic pancreatitis) each-consisting of 5 animals.
Control 1: the reproduction of pancreatitis and introduction of a physiological solution without introducing the drug; two groups (acute and chronic pancreatitis) each consisting of 5 animals.
Control 2: administering the drug to 5 intact animals, i.e. without damage of the pancreas.

After killing the animals, organs of the abdominal cavity were revised, and dynamic morphological investigations were carried out.

The proposed model of experimental pancreatitis, the chemical effect of acetic acid on the pancreas tissue enables a study of the influence of the drug on the "acute" and "chronic" stage of the development of pancreatitis. The autopsy of the animals on the 5^{th} and 12^{th} day after the reproduction of experimental pancreatitis and revision of organs of the abdominal cavity showed that only in one rat (10%) among the animals injected with the drug the inflammatory phenomena were more pronounced as compared to other animals of the group (9 animals, 90%). The damage to the pancreas is reduced by 90%.

Acute pancreatitis. Autopsy of the animals on the 5^{th} day after the reproduction of pancreatitis.

Control: acute pancreatitis, without treatment pancreas is edematic, it is closely bound to the surrounding tissues, numerous necroses of the pancreas tissue are visible, hyperemia of surrounding tissues. It is impossible to separate the pancreas.

Experiment: treatment with the drug - pancreas is edematic. Small single zones of necrosis. The pancreas tissue is closely bound to the surrounding tissues, but easily separated without injuries and bleedings.

Chronic pancreatitis. Autopsy of the animals on the 12^{th} day after the reproduction of pancreatitis. Revision of the abdominal cavity: in the group of animals without administering the preparation interintestinal abscesses remain. Necrosis of soft tissues of the stitch. Hyperemia of organs of the abdominal cavity. The pancreas is bound to the surroundings organs and tissues.

The group of animals after the administration of the drug: There are no foci of necrosis, intestine loops lie freely. The pancreas is easily separated. Hyperemia of organs of the abdominal cavity, hemorrhage is absent when touching.

Control 12 days after the pancreatitis reproduction, without treatment. Single necroses in the pancreas tissues. Hyperemia of the surrounding tissues. The separation of the pancreas is difficult; local hemorrhage may be increased.

Experiment: chronic pancreatitis, twelve days after the pancreatitis reproduction, drug introduction, no abscesses are observed in the revision of the abdominal cavity, the pancreas and surrounding tissues are not bound. The pancreas is easily separated. The tissue is edematic, tuberous. There are no necrosis foci, hyperemia of the pancreas and surrounding organs is preserved.

## Claims

1. A drug for use in the prophylaxis and treatment of acute and chronic pancreatitis containing a mixture of different polyprenyl phosphates and polyprenyl pyrophosphates with a number of isoprene units from 7 to 30 wherein the mass ratio of polyprenyl phosphates to polyprenyl pyrophosphates in the mixture is 9:1.

## Patentansprüche

1. Arzneimittel zur Verwendung in der Prophylaxe und Behandlung von akuter und chronischer Pankreatitis, enthaltend ein Gemisch aus verschiedenen Polyprenylphosphaten und Polyprenylpyrophosphaten mit Isopreneinheiten in einer Anzahl von 7 bis 30, wobei das Massenverhältnis der Polyprenylphosphaten zu den Polyprenylpyrophosphaten in der Mischung 9:1 beträgt.

## Revendications

1. Médicament pour une utilisation dans la prophylaxie et le traitement de la pancréatite aiguë et chronique, contenant un mélange de différents phosphates de polyprényle et pyrophosphates de polyprényle avec un nombre de motifs isoprène compris entre 7 et 30, dans lequel le rapport en masse des phosphates de polyprényle aux pyrophosphates de polyprényle dans le mélange est de 9:1.
